# EUROPEAN PATENT SPECIFICATION

(11) **EP 1 351 056 B1**
(45) Date of publication and mention of the grant of the patent: **03.01.2007**
(21) Application number: 03007789.5
(22) Date of filing: 04.04.2003
(51) Int. Cl.: G01N 33/543

(54) **Specific bonding analysis method and tube device therefor**
Analyseverfahren für spezifische Bindungen und Vorrichtung dafür
Procédé d'analyse de liaison spécifique et dispositif correspondant

(30) Priority: 05.04.2002 JP 2002103998; 25.02.2003 JP 2003047982
(43) Date of publication of application: 08.10.2003
(73) Proprietor: MATSUSHITA ELECTRIC INDUSTRIAL CO., LTD., Kadoma-shi, Osaka 571-8501 (JP)
(72) Inventor: Kawamura, Tatsurou, Kyotanabe-shi, Kyoto 610-0351 (JP)
(74) Representative: TBK-Patent

(56) References cited:
- EP-A- 0 310 862
- US-A- 4 366 241
- US-A- 5 624 809
- US-A- 5 985 675

## Description

### BACKGROUND OF THE INVENTION

The present invention relates to a specific bonding analysis method of conducting qualitative or quantitative analysis of an analyte in a sample.

Recently, with establishment of domestic and local care service and increase in urgent clinical examinations and the like, there is an increasing desire for development of a specific bonding analysis method by which measurement can be performed quickly, simply and correctly even by those who are not specialist in clinical examinations.

As the specific bonding analysis method, there are known a lot of methods such as an immunoassay applying an antigen-antibody reaction, a receptor assay using a receptor, a nucleic acid probe assay using hybridization of complementary nucleic acid sequence, and the like. These specific bonding analysis methods are frequently used, because of their high specificity, in wide fields typically including clinical examinations.

Further specifically, a chromatograph analysis method which is a kind of immunoassay is mentioned. In this chromatograph analysis method, for example, the presence or absence of an analyte in a sample is analyzed by utilizing a fact that a liquid sample is allowed to contact with a matrix made of a porous carrier or a fine particle-filled type carrier containing an immobilized specific bonding substance and the liquid sample flows out along the matrix by permeation force due to capillary phenomenon (Japanese Patent Nos. 2504923 and 2667793, and Japanese Patent Examined Publication (JP-B) No. 7-78503, and Japanese Patent Laid-Open Publication (JP-A) Nos. 10-73592 and 8-240591).

Specifically, a specific bonding reaction is caused between an analyte and a specific bonding substance labeled by a labeling material capable of being detected optionally by naked eyes, optical methods or the like. Then, the specific bonding substance specifically bonded with the analyte is allowed to bond to a bonding material fixed in the matrix, and the presence or absence of the analyte in a sample is finally analyzed depending on the amount of labels fixed in the matrix.

This chromatograph method is advantageous from the standpoints of measurement sensitivity and measurement time since a large amount of specific bonding substance can be insolubilized due to large surface area of a carrier in a matrix and since the impact frequency between reactive molecules capable of causing a specific bonding reaction is larger as compared with the case of reaction in liquid phrase.

In the above-mentioned conventional chromatograph method, it is necessary to use, as the matrix, a water-absorbing material capable of developing and transferring a liquid sample by capillary phenomenon. As this water-absorbing material, for example, glass fiber filter paper, cellulose membrane, nitrocellulose membrane, nylon membrane and the like are listed. These are used in the form of porous material having a pore diameter of about 1 to 50 im.

In particular, nitrocellulose is excellent since it has an ability of bonding to a large amount of a protein such as an antibody even if it is not previously sensitized. Further, nitrocelluloses having various pore diameters are available and the flow velocity of a sample can also be selected by using this.

However, though the matrix material as described above is made of a fibrous material, it is difficult to control pore diameter and surface hydrophilicity with good reproducibility in production thereof. The average value and distribution condition of the pore diameter and the hydrophilicity of the fibrous surface exert a significant influence on the developing and transferring velocity of a sample, namely, on the flow rate. Time during which a specific bonding reaction is occurring depends significantly on this flow rate, a measured value varies also depending on change in the flow rate. Namely, since the measured value is influenced with high sensitivity by the properties of the matrix material, measuring precision depends on the production precision of the matrix material.

It is difficult to improve this production precision of the matrix material to a level of securing sufficient precision of a quantitative measurement. Therefore, there has been a problem that a process of selecting a matrix material becomes necessary, leading to increase in cost. Further, since the range of pore diameter and the production precision thereof are limited, the selection width of the flow rate of a sample is also limited.

In correspondence to these facts, the specification of Japanese Patent Application No. 2001-322447 describes a specific bonding analysis method capable of easily controlling the flow rate in a wider range and having high production reproducibility of the flow rate, and a specific bonding analysis device used for this. However, with the content disclosed in the specification of Japanese Patent Application No. 2001-322447, a component not bonded to a second specific bonding substance sometimes remains on a detection part, and this remaining component sometimes contains a labeling material. Therefore, this remaining labeling material at the detection part is sometimes interposed on a detection signal. That is, the background of the detection signal increases due to the remaining labeling material, and resultantly, a S/N of the measurement sometimes decreases.

Accordingly, an object of the present invention is to provide a specific bonding analysis method in which a flow rate can be controlled in a wider range and having higher production reproducibility of the flow rate is high and, further, an increase in the background of the detection signal can be avoided, and a specific bonding analysis device used for this. According to the present invention, the selection width of the sample flow rate can be enlarged, and even on production level, the flow rate can be reproduced with high precision. Further, according to the present invention, a specific bonding analysis device of high precision can be realized at low cost since an increase in the background of the detection signal can be avoided.

US Patent No. 4,366,241 is directed to a method and an apparatus which are provided for performing immunoassays, wherein a bibulous immunosorbing zone, serving as an entry for the sample and the reagent solutions, and which contains a detection zone, is in a liquid-receiving relationship with a liquid absorbing zone, which serves to draw liquid through the immunosorbing zone by capillary force. The control of the flow-rate of the liquid may be conducted by the composition, construction, size and shape of both zones.

US Patent No. 5,985,675 discloses a test device for the detection of an analyte, wherein the test strip comprises a support strip with a plurality of sequential contacting liquid sample permeable zones extending from the first to the second end, which zones permit the lateral capillary flow of the liquid from one end to the other.

In EP-A-0 310 862, a flow through test device is described, comprising a porous support having upper and lower surfaces and a test area on its upper surface, which further includes an absorptive layer in fluid communication with a porous support, allowing the unbound analyte and tracer to flow from the test area to the absorptive layer.

US Patent No. 5,624,809 discloses a device for immunochromatographic analysis, wherein a first bibulous zone has non-diffusively bound thereto a reagent interacting with a component of a liquid. The liquid traverse all of the first zone and migrate to a second bibulous zone by capillary migration.

### BRIEF SUMMARY OF THE INVENTION

For solving the above-mentioned problems, the present invention provides a specific bonding analysis method defined according to claim 1.

In the above specific bonding analysis method, it is preferred that a duration time of the specific bonding reaction is controlled by controlling a passing velocity (flow rate) of the sample through the detection part.

The passing velocity may be expressed as transferring velocity, flow rate or the like.

It is preferred that the passing velocity of the sample through the detection part is controlled by controlling the distance and cross-sectional area of a part communicating with the outer atmosphere of the space forming part.

It is preferred that the passing velocity of the sample through the detection part is controlled by placing a first gas permeable member at a part communicating with the outer atmosphere of the space forming part.

Here, the term "part communicating with the outer atmosphere of the space forming part" means a part other than the first opening connected with the space forming part and is corresponded to a position, where air extruded by the transferring sample due to capillary phenomenon can passes through.

It is preferred that the liquid transportation force by capillary phenomenon is controlled by placing a second gas permeable member having a water absorbing property exerted by capillary phenomenon at the downstream from the detection part side along the transferring direction of the sample.

It is preferred that the specifically bonding analysis method comprises the steps of;
(A) bonding a first specific bonding substance capable of being specifically bonded to the analyte, which is labeled with a detectable labeling material, to the analyte,
(B) bonding a second specific bonding substance capable of being specifically bonded to the analyte, which is substantially immobilized on the detection part, to the analyte,
(C) measuring the strength of a signal generated in the detection part and derived from the labeling material; and
(D) qualifying or quantifying the analyte in the sample based on the strength measured in the step (C).

It is preferred that after a predetermined volume of the sample is adhered to the sample adhering part, a component not bonded to the second specific bonding substance in the step (B) is transferred to the downstream side from the detection part in the space forming part along the transferring direction of the sample by capillary phenomenon.

It is preferred that in the step (C), the first specific bonding substance and the second specific bonding substance are bonded via the analyte.

It is preferred that the first specific bonding substance is held on a contact surface of the space forming part in contact with the sample between the sample adhering part and the detection part, and the first specific bonding substance is mobilized in the wet condition with the adhered sample on the contact surface and transferred to the detection part.

The above-mentioned signal can be based on coloring, fluorescence or luminescence.

And, it is preferred that at least one of the first specific bonding substance and the second specific bonding substance is an antibody.

It is preferred that the labeling material is a particle containing metal sol, dye sol or fluorescent substance or a colored latex particle.

Further, the present invention relates to a specific bonding analysis device defined according to claim 12.

It is preferred that the above specific bonding analysis device comprises a first gas permeable member placed at a part communicating with the outer atmosphere of the space forming part.

It is preferred that the means is a second gas permeable member having a water absorbing property exerted by capillary phenomenon and placed at the downstream side from the detection part along the transferring direction of the sample.

It is preferred that the first specific bonding substance is held on a contact surface of the space forming part in contact with the sample between the sample adding part and the detection part, and the first specific bonding substance is mobilized in the wet condition with the adhered sample on the contact surface and transferred to the detection part.

It is preferred that the space forming part is constituted of two flat plates and a spacer regulating the interval of the flat plates, the detection part is provided on the flat plate, and a signal derived from the specific bonding reaction is detected via the flat plate.

While the novel features of the invention are set forth particularly in the appended claims, the invention, both as to organization and content, will be better understood and appreciated, along with other objects and features thereof, from the following detailed description taken in conjunction with the drawings.

### BRIEF DESCRIPTION OF THE SEVERAL VIEW OF THE DRAWING

Fig. 1 is a schematic sectional view showing the constitution of a specific bonding analysis device according to one embodiment of the present invention.
Fig. 2 is a schematic view of the above-mentioned specific bonding analysis device seen from the Z direction shown in Fig. 1.
Fig. 3 is a schematic sectional view showing the constitution of a specific bonding analysis device according to another embodiment of the present invention.
Fig. 4 is a schematic view of the above-mentioned specific bonding analysis device seen from the Z direction shown in Fig. 3.
Fig. 5 is an exploded perspective view of a specific bonding analysis device according to further another embodiment of the present invention.
Fig. 6 is an integrated perspective view of the specific bonding analysis device shown in Fig. 5.

### DETAILED DESCRIPTION OF THE INVENTION

The present invention relates to a specific bonding analysis method defined according to claim 1.

Here, the liquid transportation force means an ability to transfer liquid by capillary phenomenon. Larger liquid transportation force means that the hydrophilicity of the surface of a space forming part in contact with liquid is higher and the liquid retaining amount is large. Therefore, if the liquid transportation force by capillary phenomenon exerted in the space forming part is made larger in the downstream side from the above-mentioned detection part along the transferring direction of the sample, when the liquid reaches the detection part, the whole liquid is transferred to the downstream side (e.g. upward in the vertical direction). Here, it is necessary that the amount of the adhered sample is not more than the retaining amount of liquid in the downstream side.

Further, the present invention also relates to a specific bonding analysis device used in such a specific bonding analysis method. This specific bonding analysis device is defined according to claim 12.

Embodiments of the present invention will be illustrated in detail below referring to drawings.

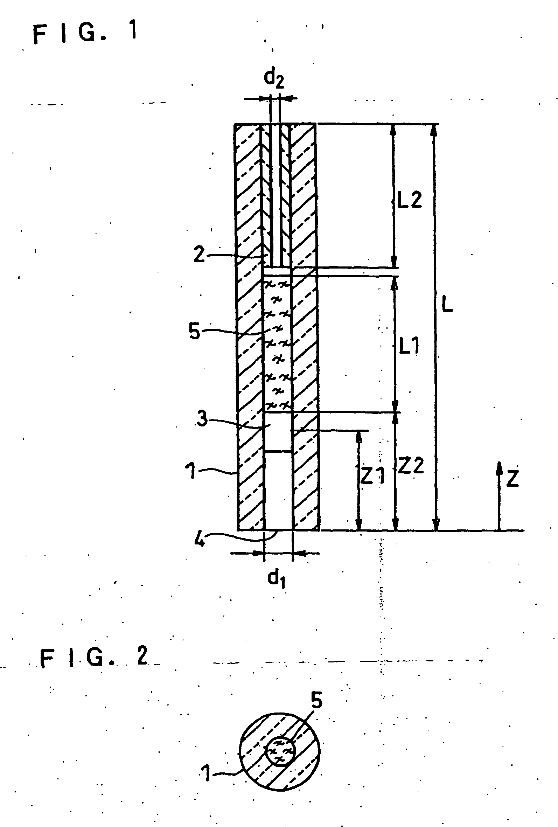

Fig. 1 is a. schematic sectional view showing the constitution of a specific bonding analysis device according to one embodiment of the present invention. Fig. 2 is a schematic view of the above-mentioned specific bonding analysis device seen from the Z direction shown in Fig. 1.

For example, this device is comprised of a first capillary tube 1 made of glass having an internal diameter (d1) of 5 mm and a length (L) of 30 mm, which constitutes a space forming part, and a second capillary tube 2 made of glass having an internal diameter (d2) of 0.5 mm, a length (L2) of 3 mm and an outer diameter of about 5 mm, which plays a role as a ventilation resistance controlling means.

As shown in Fig. 2, the second capillary tube 2 is inserted in the first capillary tube 1. Here, the outer surface of the second capillary tube 2 and the inner surface of the first capillary tube 1 are closely contacted and adhered, and air cannot permeate substantially between these tubes. The second capillary tube 2 and the first capillary tube 1 are closely and air-tightly bonded to each other by an adhesive, for example.

Inside of the first capillary tube 1, a detection part 3 is formed. This detection part 3 is formed by immobilizing or fixing a second specific bonding substance as a bonding material on the inner wall of the first capillary tube 1. This detection part 3 is positioned at a part having a distance (Z1) of about 2 mm from an opening part 4 of the first capillary tube 1 (at the side where the second capillary tube 2 is not inserted), and the length of the detection part 3 in the Z direction is about 1 mm. Z1 indicates a distance from the end of the opening part 4 to the center of the detection part 3.

Inside of the first capillary tube 1, a water-absorbing member 5 is inserted, which is a gas permeable member obtained by processing glass fiber filter paper GA200 (manufactured by TOYO KABUSHIKI KAISYA) into size of a diameter of 5 mm and a length (L2) of 20 mm. One end of the water-absorbing member 5 is positioned at a distance (Z2) of about 3 mm from the opening part 4 in the first capillary tube 1, and in contact with the detection part 3. In the present embodiment, the opening part 4 plays a role as a sample adhering part.

The specific bonding analysis device shown in Fig. 1 is placed such that the opening part 4 faces downward and the length direction of the device is substantially vertical to the horizontal direction, and the opening part 4 is contacted with the surface of a sample. In other words, the sample is adhered to the opening part 4. In this constitution, the liquid surface (level) of the sample shifts upward in the Z direction by capillary phenomenon. Namely, the liquid level of the sample shifts (rises). In this case, the liquid level of the sample shifts until the vertical direction component of the surface tension of the sample and the gravity applied on the risen sample are balanced. The gravity is one applied on the pillar-like portion of the risen sample in the first tube 1, in other words.

When the second capillary tube 2 and the water-absorbing member 5 were removed from the specific bonding analysis device shown in Fig. 1 and an aqueous solution such as urine was used as a sample in an atmosphere of ordinary atmospheric pressure and room temperature, the shift (rise) distance of the liquid level of the sample was about 6 mm. In this case, the liquid level shifted by 6 mm in about 2 to 3 seconds and stopped stationarily.

However, when only the water-absorbing member 5 was removed from the device shown in Fig. 1 and an aqueous solution such as urine was used as the sample in an atmosphere of ordinary atmospheric pressure and room temperature, the shift (rise) distance Z was about 6 mm. In this case, the liquid level shifted by 6 mm in about 30 seconds and stopped stationarily. The reason for this is as described below

With the transfer of the sample toward the upper direction by capillary phenomenon, air is pushed by a sample and, therefore, air also transferred. Here, if the opposite side of the opening part 4 of the first capillary tube 1 is sealed completely, air is compressed and pressure increases in the first capillary tube 1. Then, a difference between the pressure of this compressed air and atmospheric atmosphere, namely, an increased pressure component is further added to the gravity and, therefore, the sample stops stationarily without rising as much as 6 mm. While, if the opposite side of the opening part 4 of the first capillary tube 1 is not sealed completely, the compressed air leaks gradually by the transfer of the sample and, therefore, equivalent condition to atmospheric pressure is finally obtained and the sample rises by 6 mm and stops stationarily.

However, a duration time until the stationary stop increases as compared with a case where the opposite side of the opening part 4 of the first capillary tube 1 is opened completely (in the condition without second capillary tube 2). In other words, the transferring velocity of the sample decreases. The degree of this decrease in the transferring velocity depends on the ventilation resistance of the opposite side of the opening part 4 of the first capillary tube 1. Since this ventilation resistance changes depending on the presence or absence of the second capillary tube 2, the transferring velocity also changes depending on the presence or absence of the second capillary tube 2. Specifically, when there is no second capillary tube 2, the ventilation resistance decreases and the transferring velocity increases as compared with the presence of the second capillary tube 2.

This fact teaches that the transferring velocity of the sample can be controlled by controlling the ventilation resistance of the opposite side of the opening part 4 of the first capillary tube 1. Here, as the internal diameter (d2) of the second capillary tube 2 is smaller, in other words, as the sectional area of the interior space (space forming part) is smaller, the ventilation resistance is larger. On the contrary, as the length of the second capillary tube 2 is larger, the ventilation resistance increases more. For example, when the internal diameter (d2) and the length (L) of the second capillary tube 2 are 1.0 mm and 3 mm respectively, the sample rose by 6 mm in 7 to 10 seconds and stopped stationarily. When the internal diameter (d2) and the length (L) of the second capillary tube 2 are 0.5 mm and 2 mm respectively, the sample rose by 6 mm in 20 to 25 seconds and stopped stationarily.

The above-mentioned transferring velocity is a one in the case of no water-absorbing member 5. When the water-absorbing member 5 is inserted, the ventilation resistance naturally increases contrarily. And, the transferring velocity decreases as compared with the above-mentioned case even if the presence or absence of the second capillary tube 2 and the internal diameter and length of the second capillary tube 2 are under the same conditions as described above. However, when the constitution as shown in Fig. 1 is adopted, one end of the water-absorbing member 5 is positioned at a distance (Z2) of about 3 mm from the opening part 4 in the first capillary tube 1. Therefore, the liquid level of the sample reaches one end of the water-absorbing member 5 before rising by 6 mm. The sample is then absorbed in the water-absorbing member 5.

Next, the volume of the sample adhered to the sample adhering part will be described. When the volume of the sample is not lower than a volume necessary for the rising of the liquid level by 6 mm, the liquid level reaches to one end of the water-absorbing member 5, and the sample is absorbed. Here, it is necessary that all components, which are not bonded to the second specific bonding substance on the detection part 3, are absorbed in the water-absorbing member 5 such that the components do not remain on the detection part 3. Therefore, it is necessary that the volume of the sample, which is adhered to the sample adhering part, is not more than the maximum volume of the sample capable of being absorbed in the water-absorbing member 5.

Specifically, the maximum volume of the sample, which the water-absorbing member 5 having the above-mentioned structure can absorb, was about 0.2 ml or more. The volume of the above-mentioned sample necessary for the rising of the liquid level of the sample by 6 mm was about 0.12 ml. Therefore, when the volume of the sample was in the range from 0.12 to 0.2 ml, it was possible that all components in the sample not bonded to the second specific bonding substance on the detection part 3 could be absorbed in the water-absorbing member 5 and did not remain on the detection part 3. In the present invention, one end (lower end) of the water-absorbing member 5 is placed at a position, where the liquid level of the sample shifting by capillary phenomenon can reach in general. Further, the volume of the sample to be adhered is not lower than a volume, which enables the sample to reach the lower end of the water-absorbing member 5, and not more than a maximum volume, which the water-absorbing member 5 can absorb.

As described above, by controlling the ventilation resistance on the opposite side of the opening part 4 of the first capillary tube 1, the velocity of the sample passing through the detection part 3 can be controlled and the duration time of the specific bonding reaction, during which the specific bonding reaction is occurring, can be controlled. Therefore, the strength of a signal reflecting the specific bonding reaction can also be controlled, and sensitivity and concentration ranges in analysis can be set freely. Further, liquid transportation force by capillary phenomenon exerted in the first capillary tube 1 can be made larger in the downstream side from the detection part along the transferring direction of the sample, by the presence of the water-absorbing member 5. Therefore, it is possible not to make components not bonded to the second specific bonding substance remain on the detection part, an increase in the background of the detection signal can be avoided, and an decrease in the S/N of the signal can be prevented. The above-described numerical values are obtained when the device is placed such that the opening part 4 is directed downward and the length direction of the device is substantially vertical to the horizontal direction. Other positions, orientations or arrangements than the above one can be appropriately employed experimentally by those skilled in the art, though the above-mentioned numerical values may vary.

Further, the transferring velocity can be controlled also by controlling the ventilation resistance of the opposite side of the opening part by the ventilation resistance of the water-absorbing member 5 itself. Here, the ventilation resistance can be controlled by selecting the density, length and the like of the glass fiber filter paper GA200, while securing a necessary water-absorbing ability. Namely, the control of the transferring velocity can be realized by allowing the water-absorbing member 5 to manifest a water-absorbing effect and a ventilation resistance controlling effect.

The present invention will be illustrated more specifically below using examples, but the scope of the invention is not limited to them.

### EXAMPLE 1 and COMPARATIVE EXAMPLE 1

In the present example, human chorionic gonadotropin (hCG) in urine was analyzed as an analyte using the above-mentioned specific bonding analysis device according to the present invention shown in Fig. 1.

As the first specific bonding substance and the second specific bonding substance, an anti-hCG monoclonal antibody capable of participating in a sandwiching reaction with hCG was used. As the labeling material, a gold collide was used. Here, since the colored particle such as the gold collide was fine, it was possible to concentrate the labeled portion in a small district or volume in the detection part. Further, it was possible to correctly conduct the qualification or quantification of hCG by using a signal derived from a reaction, to which the gold colloid as the labeling material for the first specific bonding substance contributed, in the detection part 3. The inner wall of the first capillary tube 1 was blocked by passing an aqueous dispersion of skim milk through the first capillary tube 1.

First, a mixed solution containing urine and the anti-hCG monoclonal antibody labeled with the gold colloid was prepared, and this mixed solution was used as a sample. In the sample under this condition, hCG as the analyte was bonded to the anti-hCG monoclonal antibody labeled with the gold colloid. This sample was adhered in an amount of about 0.15 ml to the opening part 4 as the sample adhering part, and then the sample rose by capillary phenomenon and passed through the detection part 3. After about 1 minute passed, all liquid components were apparently absorbed in the water-absorbing member 5 and there remained apparently no liquid component on the detection part 3.

In this case, the analyte in the adhered sample was specifically bonded with the second specific bonding substance. By this, the analyte was immobilized on the detection part 3 via the second specific bonding substance. Namely, the anti-hCG monoclonal antibody as the first specific bonding substance, labeled with the gold colloid was bonded to the anti-hCG monoclonal antibody as the second specific bonding substance immobilized on the detection part 3 via hCG as the analyte. By this, the detection part 3 was colored depending on the concentration of the analyte, hCG. Here, on the detection part 3, there remained no gold colloid components not specifically bonded to the second specific bonding substance and, therefore, a contrast was by far larger as compared with the case where the water-absorbing member 5 was not present.

The hCG was added to control urine having a hCG concentration of substantially zero for management of measuring precision to prepare samples of various concentrations. Using these samples, the degree of coloration by the gold colloid in the detection part 3 was confirmed based on the above-mentioned principle. The hCG concentrations of the samples were 0(IU/L), 3(IU/L), 10(IU/L), 30(IU/L), 100(IU/L), 300(IU/L), 1000(IU/L), 3000(IU/L) and 10000(IU/L), respectively. As a result, coloration could be confirmed when the samples having a hCG concentration of 10(IU/L) or more were used.

Next, the same experiment was conducted also in the case omitting the water-absorbing member 5. In this case, the sample rose by capillary phenomenon, and the upper surface (level) of the sample shifted by about 5 mm in about 30 to 35 seconds and stopped stationarily. In this case, coloration could be confirmed when the samples having a hCG concentration of 300(IU/L) or more were used.

Further, the same experiment was conducted also in the case omitting the water-absorbing member 5 and the second capillary tube 2. In this case, the sample rose by capillary phenomenon, and the upper surface (level) of the sample shifted by about 5 mm in about 2 to 3 seconds and stopped stationarily. In this case, coloration could be confirmed only when the sample having a hCG concentration of 1000(IU/L) was used.

As described above, according to a specific bonding analysis device of the present invention, the velocity of the sample passing through the detection part 3 could be controlled by controlling the ventilation resistance of the opposite side of the opening part 4 of the first capillary tube 1. Further, liquid transportation force by capillary phenomenon exerted in the first capillary tube 1 could be made larger in the downstream side from the detection part along the transferring direction of the sample by the presence of the water-absorbing member 5. Therefore, components not bonded to the second specific bonding substance did not remain on the detection part, an increase in the background of the detection signal could be avoided and a decrease in the S/N of the signal could be prevented By this, control of the strength of a signal and the minimum detection concentration by an improvement in the S/N of the signal could be improved.

In this example, the first specific bonding substance labeled with gold colloid was mixed with urine before the adhesion, however, it may also be permissible that this first specific bonding substance is held in the dry condition between the detection part 3 and the opening part 4 as the sample adhering part. By this, urine itself can be adhered to the opening part 4 and analyzed. In this case, the first specific bonding substance held in the dry condition becomes wet by the liquid sample urine and can transfer freely, and the analyte and the first specific bonding substance transfers to the detection part 3 under the condition that they are bonded to each other, thus, coloration can be generated in the detection part 3 likewise corresponding to the concentration.

### EXAMPLE 2

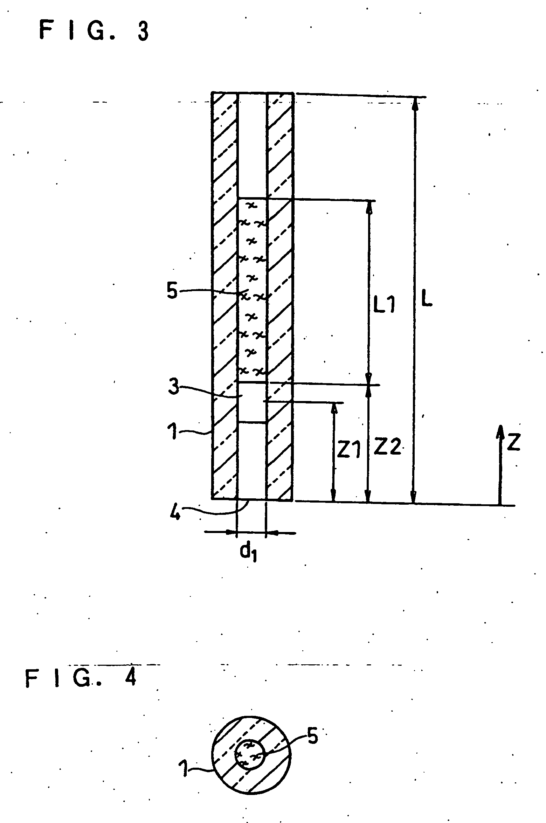

Next, human chorionic gonadotropin (hCG) as an analyte in urine was analyzed using a specific bonding analysis device according to the present invention shown in Fig. 3. Fig. 3 shows a schematic sectional view showing the constitution of a specific bonding analysis device according to another embodiment of the present invention. Fig. 4 shows a schematic view of the above-mentioned specific bonding analysis device seen from the Z direction shown in Fig. 3.

In Fig. 3, the first capillary tube 1, the detection part 3 and the opening part 4 as the sample adhering part were as the same as those used in Fig. 1. However, the second capillary tube 2 was not present, and the length L1 of the water-absorbing member 5 was changed to control the ventilation resistance. In other words, a water-absorbing member was allowed to exert also a function as a gas permeable member.

In this example, a water-absorbing member 5 obtained by processing glass fiber filter paper GA200 (manufactured by Toyo K.K.) into size of a diameter of 5 mm and a length (L2) of 20 mm as in Example 1, and a water-absorbing member 5' obtained in the same manner into size of a diameter of 5 mm and a length of 25 mm were used. Other than this, the same constitution as in the specific bonding analysis device used in Example 1 was adopted. Since the water-absorbing member 5' had a larger volume than the water-absorbing member 5, the water-absorbing ability and ventilation resistance of this were also larger than those of the water-absorbing member 5.

First, with the device shown in Figs. 3 and 4 using the water-absorbing member 5, also in this example, a mixed solution containing urine and the anti-hCG monoclonal antibody labeled with the gold colloid as in Example 1 was prepared, and this mixed solution was used as the sample and adhered to the opening part 4. This sample was adhered in an amount of about 0.15 ml to the opening part 4 as the sample adhering part, the sample rose by capillary phenomenon and passed through the detection part 3. After about 30 to 40 seconds passed, all liquid components were apparently absorbed in the water-absorbing member 5 and there remained apparently no liquid component on the detection part 3.

Also in the same manner as in Example 1, hCG was added to control urine having a hCG concentration of substantially zero for management of measuring precision to prepare samples of various concentrations, and the degree of coloration by the gold colloid in the detection part 3 was confirmed. The hCG concentrations of the samples were 0(IU/L), 3(IU/L), 10(IU/L), 30(IU/L), 100(IU/L), 300(IU/L), 1000(IU/L), 3000(IU/L) and 10000(IU/L), respectively. As a result, coloration could be confirmed when the samples having a hCG concentration of 30(IU/L) or more were used.

Next, in the device shown in Figs. 3 and 4 using the water-absorbing member 5' having changed length, the sample was adhered in an amount of about 0.15 ml to the opening part 4 as the sample adhering part. The sample rose by capillary phenomenon and passed through the detection part 3. After about 50 to 60 seconds passed, all liquid components were apparently absorbed in the water-absorbing member 5 and there remained apparently no liquid component on the detection part 3. Further, in the same manner as described above, hCG was added to control urine having a hCG concentration of substantially zero for management of measuring precision to prepare samples of various concentrations, and the degree of coloration by the gold colloid in the detection part 3 was confirmed.

The hCG concentrations of the samples were 0(IU/L), 3(IU/L), 10(IU/L), 30(IU/L), 100(IU/L), 300(IU/L), 1000(IU/L), 3000(IU/L) and 10000(IU/L), respectively. As a result, coloration could be confirmed when the samples having a hCG concentration of 10(IU/L) or more were used.

As described above, by changing the length of the water-absorbing member 5, the ventilation resistance could be changed, the transferring velocity could be controlled, and the degree of coloration at each concentration could be controlled.

As described above, according to a specific bonding analysis device of the present invention, the strength of a signal could also be controlled by controlling the ventilation resistance of the opposite side of the opening part 4 of the first capillary tube 1 by a water-absorbing member. Here, the ventilation resistance can be controlled by controlling the density, length and the like of glass fiber filter paper GA200 while securing a necessary water-absorbing ability. Namely, it could be realized by allowing the water-absorbing member 5 to exert a water-absorbing effect and a ventilation resistance controlling effect.

### EXAMPLE 3

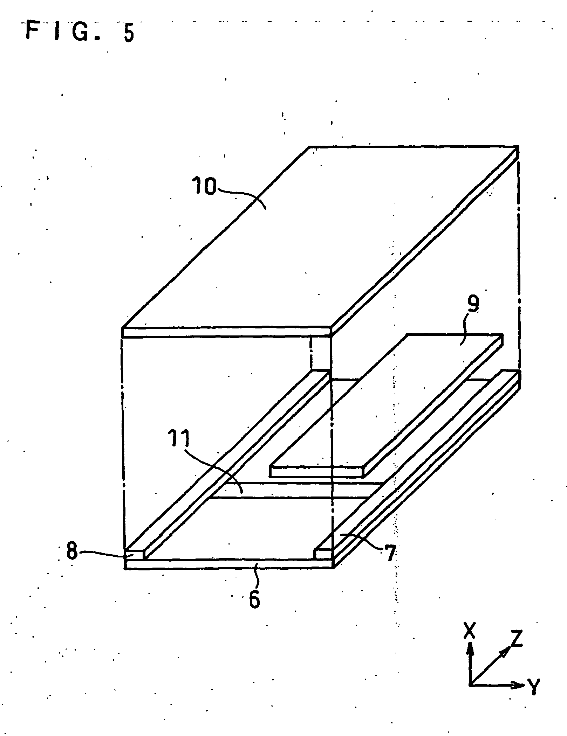

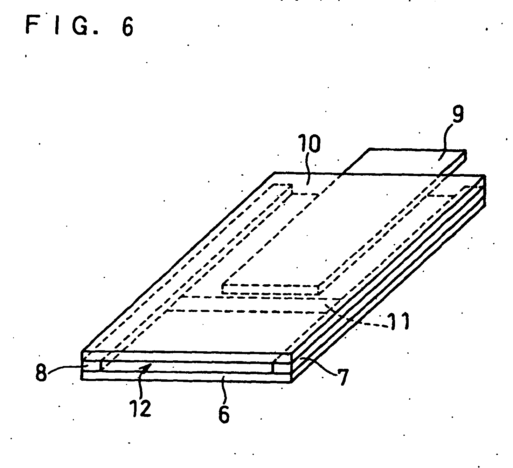

In the present example, a specific bonding analysis device shown in Figs. 5 and 6 was used. Fig. 5 shows an explosion perspective view of another specific bonding analysis device according to the present invention. As shown in Fig. 5, this specific bonding analysis device was produced by using a substrate 6 made of glass or resin, spacers 7 and 8 made of glass, resin, metal or the like (thickness in the x direction was about 50 im), a water-absorbing member 9 made of glass fiber filter paper GA200 (manufactured by TOYO KABUSHIKI KAISYA) (thickness in the x direction was about 50 im), and a transparent substrate 10 made of glass or resin. On the substrate 6, an anti-hCG monoclonal antibody capable of participating in a sandwiching reaction with hCG as the second specific bonding substance, was immobilized to form a detection part 11.

Fig. 6 shows an integrated perspective view of the specific bonding analysis device shown in Fig. 5. As shown in Fig. 6, the transparent substrate 10 was laminated with the substrate 6 via the spacers 7 and 8. By this, a space forming part was constituted inside by using the substrate 6, the spacers 7 and 8 and the transparent substrate 10. Simultaneously, a sample adhering part 12 could be constituted capable of introducing a sample into this space forming part. In this device, the ventilation resistance could be controlled by the thickness (in the x direction) of the spacers 7 and 8, the density of the glass fiber filter paper constituting the water-absorbing member 9, the space (in the y direction) between the water-absorbing member 9 and the spacers 7 and 8, and the length (in the z direction) of the above-mentioned space.

In this example, using the specific bonding analysis device according to the present invention shown in Figs. 5 and 6, human chorionic gonadotropin (hCG) in urine was analyzed as an analyte. As the first specific bonding substance and the second specific bonding substance, an anti-hCG monoclonal antibody capable of participating in a sandwiching reaction with hCG was used. And, as the labeling material, fluorescent latex was used. In this example, using a reflection absorption spectrometer, the fluorescent strength by the fluorescent latex specifically bonded to the second specific bonding substance at the detection part was measured, and the quantification of hCG could be conducted correctly. Specifically, the detection part 11 was irradiated with a light having a wavelength corresponding to the excited wavelength of the fluorescent latex, and only a light having a wavelength corresponding to the fluorescent wavelength generated at the detection part was spectroscoped and measured

After the production of the detection part 11, an aqueous dispersion of skim milk was applied on the inner wall of a space forming part constituted of the substrate 6 and the transparent substrate 10 and dried to block the above-mentioned inner wall.

First, a mixed solution containing urine and the anti-hCG monoclonal antibody labeled with a fluorescent latex was prepared, and this mixed solution was used as the sample. In the sample under this condition, hCG as the analyte was bonded to the anti-hCG monoclonal antibody labeled with the fluorescent latex. This sample was adhered in a predetermined amount to the opening part 4 as the sample adhering part. This predetermined amount means a volume of not lower than a volume, which enables the sample to reach one end of the water-absorbing member 9 by capillary phenomenon, and not more than the maximum volume, which the water-absorbing member 9 can absorb. However, for securing a quantitative property, it was necessary to make the same volume be adhered in each procedure. After the adhering, the sample rose by capillary phenomenon and passed through the detection part 11. After about 5 minutes passed, all liquid components were apparently absorbed in the water-absorbing member 9 and there remained apparently no liquid component on the detection part 11.

In this case, the analyte in the adhered sample was specifically bonded with the second specific bonding substance. By this, the analyte was immobilized to the detection part 3 via the second specific bonding substance. Namely, the anti-hCG monoclonal antibody as the first specific bonding substance, which was labeled with a fluorescent latex, was bonded to the anti-hCG monoclonal antibody as the second specific bonding substance, which was immobilized to the detection part 3 via the hCG as the analyte. By this, in the detection part 3, fluorescent generated depending on the concentration of the hCG as the analyte. Here, on the detection part 11, there remained no fluorescent latex not specifically bonded to the second specific bonding substance and, therefore, the background decreased significantly as compared with the case where the water-absorbing member 9 was not present.

The hCG was added to control urine having a hCG concentration of substantially zero for management of measuring precision to prepare samples of various concentrations, and the fluorescent strength by the fluorescent latex in the detection part 11 was measured based on the above-mentioned principle. The hCG concentrations of the samples were 0(IU/L), 3(IU/L), 10(IU/L), 30(IU/L), 100(IU/L), 300(IU/L), 1000(IU/L), 3000(IU/L) and 10000(IU/L), respectively. As a result, fluorescence could be confirmed when the sample having a hCG concentration of 10(IU/L) or more were used, and the linear property of a curve plotting the hCG concentration and the fluorescent strength could be confirmed until 1000(IU/I). Namely, a quantification property could be confirmed from 10(IU/L) to 1000(IU/I).

This quantification property could be controlled by controlling the ventilation resistance. Namely, the minimum detection concentration and the maximum concentration showing a linear property could be controlled.

As described above, according to a specific bonding analysis device of the present invention, the velocity of a sample passing through the detection part 11 could be controlled by controlling the ventilation resistance of the opposite side of the sample adhering part 12. Further, the background of the detection signal could be decreased and the S/N of the signal was improved since components not bonded to the second specific bonding substance did not remain on the detection part due to the effect of the water-absorbing member 9. According to the effect, the liquid transportation force by capillary phenomenon exerted in the space forming part could be made larger in the transferring direction side of the sample rather than the detection part 11 by the presence of the water-absorbing member 9. Thus, the control of a quantification property and the minimum detection concentration by an increase in the S/N of the signal could be improved.

In this example, the first specific bonding substance labeled was mixed with urine before the adhesion, however, it may also be permissible that this first specific bonding substance is held in the dry condition between the sample adhering part 12 and the detection part 11. Thus, urine itself can be adhered to the sample adhering part 12 and analyzed. In this case, the first specific bonding substance held in the dry condition becomes wet by the liquid sample urine and can transfer freely, and the analyte and the first specific bonding substance can transfer to the detection part 11 under the condition thath they are bonded to each other and, likewise, fluorescence can be generated corresponding to concentration.

As described above, according to the specific bonding analysis device of this example, sensitivity and concentration range in analysis could be freely set.

As described above, according to the specific bonding analysis method and the device using this of the present invention, the velocity of the sample passing through the detection part can be controlled, further, components not immobilized by the second specific bonding substance do not tend to remain on the detection part and, therefore, sensitivity and concentration range can be freely set, and sensitivity also increases. That is, the present invention is practically extremely effective.

Although the present invention has been described in terms of the presently preferred embodiments, it is to be understood that such disclosure is not to be interpreted as limiting. Various alterations and modifications will no doubt become apparent to those skilled in the art to which the present invention pertains, after having read the above disclosure.

A specific bonding analysis method by which sensitivity and concentration range in analysis can be freely set, and a device using this are provided. The intended analysis is optimized by using a specific bonding analysis device capable of controlling the velocity of a sample passing through a detection part and causing no remaining of unnecessary components on a detection part.

## Claims

1. A specific bonding analysis method comprising the steps of:
preparing a specific bonding analysis device comprising a first opening (4, 12) for adding a liquid sample containing an analyte, a space forming part exerting capillary phenomenon connected with said first opening (4, 12), said space forming part being constituted by a capillary tube, or tube flat plates and a spacer regulating the interface of the flat plates, a second opening opposite said first opening (4, 12) connected with said space forming part, the space forming part further comprising a detection part (3, 11) formed by immobilising a specific bonding substance needed for the analyte detection on the inner wall of said space forming part downstream of: said first opening (4, 12) and a water absorbing member (5, 9) which is provided in said space forming part downstream of said detection part (3, 11);
introducing said liquid sample to said first opening (4, 12) and through said detection part (3, 11) in said space forming part by capillary phenomenon, thereby causing a specific bonding reaction with said analyte;
detecting a signal derived from the specific bonding reaction to qualify or quantify said analyte; and
controlling liquid transportation force by capillary phenomenon in said space forming part by said water absorbing member (5, 9).

2. The specific bonding analysis method according to claim 1, wherein a duration time of said specific bonding reaction is controlled by controlling a passing rate of said sample through said detection part (3, 11).

3. The specific bonding analysis method according to claim 2, wherein said passing rate of said sample through said detection part is controlled by controlling the distance and cross-sectional area of a part communicating with the outer atmosphere of said space forming part.

4. The specific bonding analysis method according to claim 1, wherein liquid transportation force by capillary phenomenon is controlled by placing a second water absorbing member (5') at the downstream from said detection part (3, 11) side along the transferring direction of said sample.

5. The specific bonding analysis method according to claim 1, comprising the steps of;
(A) bonding a first specific bonding substance capable of being specifically bonded to said analyte, which is labeled with a detectable labeling material, to said analyte,
(B) bonding a second specific bonding substance capable of being specifically bonded to said analyte, which is substantially immobilized on said detection part (3, 11), to said analyte,
(C) measuring the strength of a signal generated in said detection part and derived from said labeling material; and
(D) qualifying or quantifying said analyte in said sample based on said strength measured in said step (C).

6. The specific bonding analysis method according to claim 5, wherein after a predetermined volume of said sample is adhered to said first opening (4, 12), a component not bonded to said second specific bonding substance in said step (B) is transferred to the downstream side from said detection part (3, 11) in said space forming part along the transferring direction of said sample by capillary phenomenon.

7. The specific bonding analysis method according to claim 5, wherein in said step (C), said first specific bonding substance and said second specific bonding substance are bonded via said analyte.

8. The specific bonding analysis method according to claim 1, wherein said first specific bonding substance is held on a contact surface of said space forming part in contact with said sample between said first opening (4, 12) and said detection part (3, 11), and said first specific bonding substance is mobilized in a wet condition with said adhered sample on said contact surface and transferred to said detection part (3, 11).

9. The specific bonding analysis method according to claim 1, wherein said signal is coloring, fluorescence or luminescence.

10. The specific bonding analysis method according to claim 1, wherein at least one of said first specific bonding substance and said second specific bonding substance is an antibody.

11. The specific bonding analysis method according to claim 5, wherein said labeling material is a particle containing metal sol, dye sol or fluorescent substance or a colored latex particle.

12. A specific bonding analysis device comprising:
a first opening (4, 12) for adding a liquid sample containing an analyte;
a space forming part exerting capillary phenomenon connected with said first opening (4, 12), said space forming part being constituted by a capillary tube, or two flat plates and a spacer regulating the interface of the flat plates;
a second opening opposite to said first opening connected with said space forming part, the space forming part further comprises a detection part, formed by immobilising a specific bonding substance need for the analyte detection on the inner wall of said space forming part;
and comprising
a water absorbing member (5, 9) which is provided in said space forming part downstream of said detection part;
whereby said sample is transferred to said detection part (3, 11) in said space forming part by capillary phenomenon to cause a specific bonding reaction and a signal derived from the specific bonding reaction is detected to qualify or quantify said analyte.

## Patentansprüche

1. Analyseverfahren für eine spezifische Bindung mit den Schritten:
Bereitstellen einer Analysevorrichtung für eine spezifische Bindung mit einer ersten Öffnung (4, 12) für die Zugabe einer flüssigen Probe, die ein Analyt enthält, einem raumbildenden Teil, der ein Kapillarphänomen aufweist, und mit der ersten Öffnung (4, 12) verbunden ist, wobei der raumbildende Teil aufgebaut wird durch eine Kapillarröhre oder aus zwei flachen Platten und einem Abstandshalter, der den Abstand der flachen Platten reguliert, einer zweiten Öffnung gegenüber der ersten Öffnung (4, 12), die mit dem raumbildenden Teil verbunden ist, wobei das raumbildende Teil außerdem einen Nachweisteil (3, 11) stromabwärts der ersten Öffnung (4, 12) umfasst, der durch Immobilisieren einer spezifisch bindenden Substanz gebildet wird, die für den Analytnachweis auf der inneren Wand des raumbildenden Teils benötigt wird, und mit einem Wasser-absorbierenden Element (5, 9), welches in dem raumbildenden Teil stromabwärts des Nachweisteils (3, 11) vorgesehen wird;
Einbringen der flüssigen Probe in die erste Öffnung (4, 12) und durch den Nachweisteil (3, 11) in den raumbildenden Teil durch das Kapillarphänomen, wodurch eine spezifische Bindungsreaktion mit dem Analyt verursacht wird;
Nachweis eines Signals, das aus der spezifischen Bindungsreaktion stammt, um den Analyt zu qualifizieren oder zu quantifizieren; und
Steuern der Flüssigkeitstransportkraft durch das Kapillarphänomen in dem raumbildenden Teil durch das Wasser-absorbierende Element (5, 9).

2. Analyseverfahren für eine spezifische Bindung nach Anspruch 1, wobei die Zeitdauer der spezifischen Bindungsreaktion durch Steuerung der Durchtrittsgeschwindigkeit der Probe durch den Nachweisteil (3, 11) gesteuert wird.

3. Analyseverfahren für eine spezifische Bindung nach Anspruch 2, wobei die Durchtrittsgeschwindigkeit der Probe durch den Nachweisteil durch die Steuerung des Abstands und der Querschnittsfläche eines Teils gesteuert wird, der mit der äußeren Atmosphäre des raumbildenden Teils kommuniziert.

4. Analyseverfahren für eine spezifische Bindung nach Anspruch 1, wobei die Flüssigkeitstransportkraft durch das Kapillarphänomen durch Anordnen eines zweiten Wasser-absorbierenden Elements (5') stromabwärts von der Seite des Nachweisteils (3, 11) entlang der Transferrichtung der Probe gesteuert wird.

5. Analyseverfahren für eine spezifische Bindung nach Anspruch 1 mit den Schritten:
(A) Binden einer ersten spezifisch bindenden Substanz, die spezifisch an das Analyt gebunden werden kann, welches mit einem nachweisbaren markierenden Material markiert ist, an das Analyt,
(B) Binden einer zweiten spezifisch bindenden Substanz, die spezifisch an das Analyt gebunden werden kann, welches im Wesentlichen auf dem Nachweisteil (3, 11) immobilisiert ist, an das Analyt,
(C) Messen der Stärke eines Signals, das den Nachweisteil erzeugt wird und aus dem markierenden Material stammt; und
(D) Qualifizieren oder Quantifizieren des Analyts in der Probe auf der Grundlage der in den Schritt (C) gemessenen Stärke.

6. Analyseverfahren für eine spezifische Bindung nach Anspruch 5, wobei nachdem ein vorbestimmtes Volumen der Probe an die erste Öffnung (4, 12) angeheftet ist, ein nicht an die zweite spezifisch bindende Substanz unten genannter Bestandteil in dem Schritt (B) zu der Seite stromabwärts von dem Nachweisteil (3, 11) in dem raumbildenden Teil entlang der Transferrichtung der Probe des Kapillarphänomens transferiert wird.

7. Analyseverfahren für eine spezifische Bindung nach Anspruch 5, wobei in dem Schritt (C) die erste spezifisch bindende Substanz und die zweite spezifisch bindende Substanz über das Analyt gebunden sind.

8. Analyseverfahren für eine spezifische Bindung nach Anspruch 1, wobei die erste spezifisch bindende Substanz auf einer Kontaktoberfläche des raumbildenden Teils in Kontakt mit der Probe zwischen der ersten Öffnung (4, 12) und dem Nachweisteil (3, 11) und der ersten spezifisch bindenden Substanz in einem feuchten Zustand der angehafteten Probe auf der Kontaktoberfläche immobilisiert und zu dem Nachweisteil (3, 11) transferiert wird.

9. Analyseverfahren für eine spezifische Bindung nach Anspruch 1, wobei das Signal Färbung, Fluoreszenz oder Lumineszenz ist.

10. Analyseverfahren für eine spezifische Bindung nach Anspruch 1, wobei wenigstens eine der ersten spezifisch bindenden Substanz und der zweiten spezifisch bindenden Substanz ein Antikörper ist.

11. Analyseverfahren für eine spezifische Bindung nach Anspruch 5, wobei das markierende Material ein Teilchen ist, das ein Metallsol, ein Farbstoffsol oder eine fluoreszierende Substanz oder ein gefärbtes Latexteilchen enthält.

12. Analysevorrichtung für eine spezifische Bindung mit:
einer ersten Öffnung (4, 12) für die Zugabe einer flüssigen Probe, die ein Analyt enthält;
einem raumbildenden Teil, der ein Kapillarphänomen aufweist, und mit der ersten Öffnung (4, 12) verbunden ist, wobei der raumbildende Teil aufgebaut ist durch eine Kapillarröhre oder zwei flache Platten und einem Abstandshalter, der die Grenzflächen der flachen Platten reguliert;
einer zweiten Öffnung gegenüber der ersten Öffnung, die mit dem raumbildenden Teil verbunden ist, wobei der raumbildende Teil außerdem einen Nachweisteil umfasst, der durch Immobilisieren einer spezifisch bindenden Substanz ausgebildet ist, die für den Analytnachweis auf der inneren Wand des raumbildenden Teils benötigt wird;
und mit
einem Wasser-absorbierenden Element (5, 9), welches in dem raumbildenden Teil stromabwärts des Nachweisteils vorgesehen ist;
wodurch die Probe zu den Nachweilsteilen (3, 11) in den raumbildenden Teil durch das Kapillarphänomen transferiert wird, um eine spezifische Bindungsreaktion zu verursachen, und ein aus der spezifischen Bindungsreaktion stammendes Signal wird nachgewiesen, um das Analyt zu qualifizieren oder zu quantifizieren.

## Revendications

1. Procédé d'analyse de liaison spécifique comprenant les étapes consistant à :
préparer un dispositif d'analyse de liaison spécifique comprenant une première ouverture (4, 12) pour ajouter un échantillon liquide contenant un analyte, une partie formant un espace exerçant un phénomène capillaire connectée à ladite première ouverture (4, 12), ladite partie formant un espace étant constituée par un tube capillaire, ou des plaques plates de tube et un espaceur régulant l'interface des plaques plates, une deuxième ouverture opposée à ladite première ouverture (4, 12) connectée à ladite partie formant un espace, la partie formant un espace comprenant en plus une partie de détection (3, Il) formée en immobilisant une substance de liaison spécifique requise pour la détection d'analyte sur la paroi intérieure de ladite partie formant un espace en aval de ladite première ouverture (4, 12) et un organe d'absorption d'eau de partie formant un espace (5, 9) qui est prévu dans ladite partie formant un espace en aval de ladite partie de détection (3,11) ;
introduire ledit échantillon liquide à ladite première ouverture (4, 12) et à travers ladite partie de détection (3, 11) dans ladite partie formant un espace par phénomène capillaire, entraînant ainsi une réaction de liaison spécifique avec ledit analyte ;
détecter un signal dérivé de la réaction de liaison spécifique pour qualifier ou quantifier ledit analyte ; et
contrôler une force de transport de liquide par phénomène capillaire dans ladite partie formant un espace par ledit organe d'absorption d'eau (5, 9).

2. Procédé d'analyse de liaison spécifique selon la revendication 1, dans lequel une durée de ladite réaction de liaison spécifique est contrôlée en commandant une vitesse de passage dudit échantillon à travers ladite partie de détection (3, 11).

3. Procédé d'analyse de liaison spécifique selon la revendication 2, dans lequel ladite vitesse de passage dudit échantillon à travers ladite partie de détection est commandée en contrôlant la distance et une zone transversale d'une partie communiquant avec l'atmosphère extérieure de ladite partie formant un espace.

4. Procédé d'analyse de liaison spécifique selon la revendication 1, dans lequel une force de transport de liquide par phénomène capillaire est contrôlée en plaçant un deuxième organe d'absorption d'eau (5') en aval dudit côté de partie de détection (3, 11) le long de la direction de transfert dudit échantillon.

5. Procédé d'analyse de liaison spécifique selon la revendication 1, comprenant les étapes consistant à ;
(A) lier une première substance de liaison spécifique capable d'être liée de façon spécifique audit analyte, qui est marquée avec un matériau de marquage détectable, audit analyte,
(B) lier une deuxième substance de liaison spécifique capable d'être liée de façon spécifique audit analyte, qui est essentiellement immobilisée sur ladite partie de détection (3, 11), audit analyte,
(C) mesurer la force d'un signal généré dans ladite partie de détection et dérivé dudit matériau de marquage ; et
(D) qualifier ou quantifier ledit analyte dans ledit échantillon sur la base de ladite force mesurée dans ladite étape (C).

6. Procédé d'analyse de liaison spécifique selon la revendication 5, dans lequel après qu'un volume prédéterminé dudit échantillon est adhéré à ladite première ouverture (4, 12), un composant non lié à ladite deuxième substance de liaison spécifique dans ladite étape (B) est transféré au côté aval de ladite partie de détection (3, 11) dans ladite partie formant un espace le long de la direction de transfert dudit échantillon par phénomène capillaire.

7. Procédé d'analyse de liaison spécifique selon la revendication 5, dans lequel dans ladite étape (C), ladite première substance de liaison spécifique et ladite deuxième substance de liaison spécifique sont liées à travers ledit analyte.

8. Procédé d'analyse de liaison spécifique selon la revendication 1, dans lequel ladite première substance de liaison spécifique est maintenue sur une surface de contact de ladite partie formant un espace en contact avec ledit échantillon entre ladite première ouverture (4, 12) et ladite partie de détection (3, 11), et ladite première substance de liaison spécifique est mobilisée dans une condition humide avec ledit échantillon adhéré sur ladite surface de contact et transférée à ladite partie de détection (3, 11).

9. Procédé d'analyse de liaison spécifique selon la revendication 1, dans lequel ledit signal est une coloration, une fluorescence ou une luminescence.

10. Procédé d'analyse de liaison spécifique selon la revendication 1, dans lequel au moins une de ladite première substance de liaison spécifique et de ladite deuxième substance de liaison spécifique est un anticorps.

11. Procédé d'analyse de liaison spécifique selon la revendication 5, dans lequel ledit matériau de marquage est un sol métallique, un sol de colorant ou une substance fluorescente contenant une particule ou une particule de latex coloré.

12. Dispositif d'analyse de liaison spécifique comprenant :
une première ouverture (4, 12) pour ajouter un échantillon liquide contenant un analyte ;
une partie formant un espace exerçant un phénomène capillaire connectée à ladite première ouverture (4, 12), ladite partie formant un espace étant constituée par un tube capillaire, ou deux plaques plates et un espaceur régulant l'interface des plaques plates ;
une deuxième ouverture opposée à ladite première ouverture connectée à ladite partie formant un espace, la partie formant un espace comprend en plus une partie de détection, formée en immobilisant une substance de liaison spécifique requise pour la détection d'analyte sur la paroi intérieure de ladite partie formant un espace ;
et comprenant
un organe d'absorption d'eau (5, 9) qui est prévu dans ladite partie formant un espace en aval de ladite partie de détection ;
par lequel ledit échantillon est transféré à ladite partie de détection (3, 11) dans ladite partie formant un espace par phénomène capillaire pour entraîner une réaction de liaison spécifique et un signal dérivé de la réaction de liaison spécifique est détecté pour qualifier ou quantifier ledit analyte.
